# EUROPEAN PATENT APPLICATION

(11) **EP 2 774 537 A1**
(43) Date of publication of application: **10.09.2014**
(21) Application number: 13158351.0
(22) Date of filing: 08.03.2013
(51) Int. Cl.: A61B 5/055, A61N 5/10, A61B 6/00

(54) **Patient alignment in MRI guided radiation therapy**

(71) Applicant: Imris Inc., Winnipeg, Manitoba R3T 1N5 (CA)
(72) Inventor: Winter, Jeff, Winnipeg, Manitoba R3T 1N5 (CA); Westmore, Michael, Winnipeg, Manitoba R3T 1N5 (CA); Dahan, Meir, Winnipeg, Manitoba R3T 1N5 (CA); Petropoulos, Labros, Winnipeg, Manitoba R0G 1B0 (CA); Guyot, Brendan, Winnipeg, Manitoba R2M 1E3 (CA); Jaffray, David A., Toronto, Manitoba M5G 2M9 (CA); Carlone, Marco, Toronto, Manitoba M5G 2M9 (CA); Stanescu, Teodor, Toronto, Manitoba M5G 2M9 (CA)
(74) Representative: Jostarndt, Hans-Dieter

(57) **Abstract**

Apparatus for radiation therapy combines a patient table, an MRI and a radiation treatment apparatus mounted in a common treatment room with the MR magnet movable through a radiation shielded door to an imaging position. An initial MR image and an initial X-ray image is used to generate an RT program for the patient to be carried out in a plurality of separate treatment steps. Before carrying out the procedure, a registration step is performed using a phantom by which X-ray images are registered relative to MR images to generate a transformation algorithm required to align the MR images of the part of the patient relative to the X-ray images. Prior to each separate treatment step a current MR image of the part of the patient is obtained and the transformation algorithm data is used from the current MR image is used in guiding the RT treatment step.

## Description

This invention relates to a system for patient alignment in magnetic resonance imaging guided radiation therapy which can provide an additional level of quality assurance that the treatment is being accurately applied.

### BACKGROUND OF THE INVENTION

Image-guided radiation therapy (RT) utilizes imaging to assess the position of the patient prior to radiotherapy treatment to ensure correct positioning for accurate delivery of prescribed radiation dose to cancerous tumours or other lesions, whilst minimizing the dose to healthy tissue.

External beam radiotherapy (RT) devices generally include a linear electron beam accelerator which is mounted on a gantry, which can rotate about an axis approximately parallel to the patient lying on the patient couch. The patient is treated using either an electron beam or an X-Ray beam produced from the original electron beam. The electron or X-Ray beam is focused at a target by the combination of the use of a collimator and the rotation of the beam. The patient is placed on a couch that can be positioned such that the target lesion can be located in the plane of the radiation beam as the gantry rotates.

The radiotherapy device generally includes a linear electron beam accelerator which is mounted on a gantry and which can rotate about an axis which is approximately parallel to the patient lying on the patient couch. The patient is treated using either an electron, gamma or X-Ray beam produced from the original electron beam. The beam is focused at a target by the combination of the use of a collimator and the rotation of the beam. The patient is placed on a couch which can be positioned such that the target lesion can be located in the plane of the electron beam as the gantry rotates. This patient couch is designed to adjust the position of the patient to align the targeting exactly at the isocentre of the RT system using up to six degrees of motion (x, y, z, roll, pitch, and yaw). The current couch designs employed by several manufacturers employ a cantilevered couch that enables a sufficient range of motion to treat disease sites throughout the entire body.

The objective of the radiation therapy is to target the lesion with a high dose of radiation with minimal impact on all the surrounding normal tissue. An initial treatment planning procedure is performed prior to external beam RT delivery to localize the tumour and other critical structures surrounding the tumour. This planning procedure typically involves CT imaging to identify these structures. Based on the segmented tumour and surrounding tissue structures, a set of beam orientations and collimator settings are developed through an iterative process to determine the optimal dose distribution pattern that maximizes dose to the tumour whilst minimizing dose to surrounding critical avoidance structures.

MRI is currently the optimal modality for tumour localization based on the higher soft-tissue contrast, compared with CT, and can be incorporated into the treatment planning workflow. Although MRI provides good location of the tumour for treatment planning purposes, these treatment planning images are normally collected several days prior to treatment, and, as such, may not be completely representative of tumour location on the day of treatment. To address this limitation, oncologists tend to increase the target volume ensure all the tumor tissue receives the maximum dose. The expectation is that all cells in the targeted region will receive the required RT treatment dose, and that this increased treatment target volume will lessen the impact of errors between treatment planning dose distribution, and the dose delivered to the actual region of the lesion. However, this increased treatment margin also produces collateral tissue damage that may have a significant impact of the quality of life of the patient and increase the possibility of secondary RT-induced cancer.

To mitigate the need for increased treatment margins, clinicians have employed a method referred to as image-guided external beam radiation therapy, in which an image is acquired immediately prior to RT treatment delivery. One such available solution involves completely integrating the MRI system with a linear accelerator to enable real-time imaging of the tumour during RT treatment. However, this design is complex, expensive and may involve serious compromises on the functional performance of both the MRI and linear accelerator.

The treatment planning images are typically collected days prior to the actual fractionated treatment delivery that can occur over the course of several weeks. As such, the position of the tumour in the treatment imaging plans may not be representative of the actual lesion position on each day of treatment.

The initial step in image-guided radiation therapy is to develop a treatment plan that prescribes the dose distribution patterns within the tumour and various critical tissue structures. This planning procedure is typically performed ahead of the first RT treatment fraction, and typically starts with the acquisition of a set of computed tomography (CT) images. Owing to reduced soft tissue contrast and limited functional information in CT, it is typical to collect a set of magnetic resonance (MR) and/or PET images to aid the contouring of tissue structures. Once the tumour and other adjacent critical organ structures are contoured and specific treatment margins are defined, a RT treatment planning station utilizes the x-ray attenuation information from computed tomography (CT) images to create a series of beam orientations and collimation settings to generate and optimize dose patterns throughout the treatment volume. The beam and collimation settings are then validated on the linear accelerator to ensure that the measured dose patterns match the prescribed treatment plan. Once complete, the treatment plan is ready for the first treatment fraction.

The patient is scheduled for a series of treatment fractions, so that the total dose delivered is spread across multiple days to minimize radiation side effects. On the first treatment fraction, the patient is placed on the couch and aligned using lasers and visual inspection by the radiation therapist. For image-guided radiation therapy, a room- or gantry-mounted x-ray imaging system is used to collect either 2 dimensional (2D) projection images or 3 dimensional (3D) cone-beam CT (CBCT) images. On the linear accelerator image guidance system, the radiation therapist is able to use either manual adjustments or automated image registration techniques to align the daily x-ray images with the 3D CT (or 2D digitally reconstructed radiographs) images used for treatment planning to determine the patient shifts required to bring the patient position in line with the treatment plan. These required shifts are delivered to the treatment couch, which couch adjusts the couch-top position to match the current patient position to the patient's treatment planning position.

There has been wide-spread adoption of x-ray image-guided methods to position the patient ahead of each treatment fraction; however, x-ray imaging does not provide enough soft tissue contrast required to clearly identify the tumour. This shortcoming of x-ray imaging has led to the development of MRI-guided RT systems that provide MR imaging ahead of treatment to position the patient. One such approach is to utilize a moving magnet that can be brought into the linear accelerator bunker for imaging immediately prior to treatment. Using these Daily MR images, it is possible to align the patient based on the superior soft tissue contrast in MR that enables visualization of the actual tumour.

One problem in the scenario of daily MR acquisition ahead of RT therapy, is a lack of correspondence between the MR imaging coordinate system, and the Linac coordinate system (determined by the intersection of the megavoltage (MV) beams of the linear accelerator positioned at different beam angles) due to use of a moving magnet. One approach to solve this issue is to use purely anatomical-based registration to align the MR coordinate system with the Linac coordinate system. But, this anatomical registration would have to be based on parts of the image not expected to change over time (such as the bony anatomy), which is extremely challenging and prone to errors.

Lastly, incorporation of MR data into the RT treatment requires additional checks to ensure that the correct imaging data is sent to the RT delivery software architecture. This includes ensuring that the correct patient data is transferred and that an appropriate MR imaging technique and parameters are used to acquire the images. Moreover, it is critical that if a Daily MR is collected, a corresponding treatment plan MR also exists for that patient.

IMRIS in US Serial No: 12/420,859 filed April 8, 2009 and published as Publication No 2009-0306494 published December 10th 2009 describes the integration of MRI with radiation therapy although the method to integrate the linear accelerator within the RF-shielded room is not disclosed. The arrangement provides bunker doors separating the MR system from the linear accelerator and the moving magnet.

IMRIS in WO 2010/111772 7 published October 2010 discloses a patient support system for integrating X-ray imaging with MR. This application describes a support structure that is both MR compatible and radiolucent, which is described in this invention record.

IMRIS in US Serial No: 12/792,383 filed June 2 2010 and published as Publication 2010-0329414 published December 30th 2010 describes a rotating table top, and MR/CT/RT systems angularly situated around the patient.

Bucholz et al. discloses a method to combine proton beam therapy with an MRI system in U.S. Patent 6,862,469. This method only discusses proton therapy, describes a stationary MRI, in which the beam is sent through a gap in the magnet. This application suggests that shielding methods can be used to remove magnetic and RF interference, although this is only briefly mentioned.

Dempsey in U.S. Patent Application Publication No.2005/0197564 discloses a method to deliver RT using cobalt-60 as the source of ionizing radiation with a stationary open MRI system device and a process for performing high temporal and spatial-resolution MR imaging of the anatomy of a patient during intensity modulated radiation therapy (IMRT) to directly measure and control the highly conformal ionizing radiation dose delivered to the patient for the treatment of diseases caused by proliferative tissue disorders. This invention combines the technologies of open MRI, multileaf-collimator or compensating filter-based IMRT delivery, and cobalt teletherapy into a single co-registered and gantry mounted system.

Carlone in WO/2009/155700 discloses a method to combine MRI and a radiation therapy system. This method describes an approach that exposes the linear accelerator to the magnetic field, and uses the magnet forces to direct the particles along the central axis.

Lagendijk discloses in WO/2003/008986 a method to combine MRI and radiation therapy using a global coordinate system, entitled MRI in guided radiotherapy and position verification.

Other patents describing prior art include:
US patent 6,198,957-Radiotherapy Machine including Magnetic Resonance imaging system
US patent 6,366,798-Radiotherapy machine including Magnetic Resonance Imaging system.
US Patent 6,725,078-System combining proton beam irradiation and magnetic resonance imaging.
US Patent 5,402,783-Method of Minimizing distortion to radiation isodose.
US Patent 6,419,680-CT and MRI visible index markers for stereotactic localization. In this patent application, the inventors claim that skin-based localizer markers can be used for stereotactic localization in both MRI and CT.

WO 03/008986 (Utrecht) A2 describes MRI in guided radiotherapy and position verification incorporating an independent world coordinate isocentre calibration system consisting of fiducial table MR-markers and an independent table position verification system. This patent is similar to this current IR, however, the Utrecht patent may be specific only to the hybrid MR/Linac systems. Moreover, markers in this invention are only MR visible, and not necessarily X-ray visible.

The disclosure of all of the above cited references may be referred to for further details of components and methods not specifically set out herein.

### SUMMARY OF THE INVENTION

According to one aspect of the invention there is provided a method for treating a patient with radiation therapy or a locating a patient relative to an RT treatment system for the RT treatment to occur where the method comprises:
providing a patient support table;
providing an MR imaging system for acquiring MR images of an imaging volume including a target location of the patient;
the MR imaging system including:
   an MR magnet with gradient coil operable to generate a variable magnetic field to be applied to the patient, the magnet having a cylindrical bore for surrounding the target location of the patient;
   an RF transmit coil arrangement for generating an RF pulse in a transmit stage to be applied to the subject to be imaged such that the subject generates an MR signal in response to the magnetic field and the RF pulse applied;
   a receive coil arrangement for acquiring the MR signal in a receive stage;
   and a signal processing system for receiving the MR signal for carrying out signal processing by which an image is generated;
providing a treatment apparatus for applying a radiation therapy to the target location in the patient while on the support table;
the treatment apparatus having an X-ray imaging system for obtaining an X-ray image of the patient using X-rays;
the MR magnet being mounted for movement from a position remote from the patient on the patient support table to an imaging position for cooperation with the patient on the patient support table for MR imaging;
using the MR imaging system to obtain MR images of a part of the patient to determine the target location of a treatment site in the patient;
using the X-ray imaging system of the treatment apparatus to obtain X-ray images of the part of the patient for use in guiding the RT treatment;
using an initial MR image and an initial X-ray image to generate an RT program for the patient to be carried out in a plurality of separate treatment steps
registering X-ray images taken by the X-ray imaging system relative to MR images taken by the MR imaging system to generate a transformation algorithm required to align the MR images of the part of the patient relative to the X-ray images of the part of the patient;
prior to each separate treatment step, obtaining a current MR image of the part of the patient;
and using the transformation algorithm to use data from the current MR image in guiding the RT treatment step.

Preferably the MR imaging system is used to obtain a calibration MR image of the phantom with fiducial markers prior to all treatment fractions, wherein the X-ray imaging system of the treatment apparatus is used to obtain a calibration X-ray of the phantom with fiducial markers prior to all treatment fractions and wherein the calibration X-ray images taken by the X-ray imaging system are registered relative to calibration MR images taken by the MR imaging system to generate the transformation algorithm required to align the MR coordinate system relative to the X-ray coordinate system.

Preferably prior to each day of treatment for a plurality of patients, the method includes performing a quality assurance procedure of the transformation algorithm by repeating the calibration MR and X-ray image acquisitions and updating the transformation algorithm based on the quality assurance results.

According to a further aspect of the invention there is provided a method for treating a patient with radiation therapy or locating a patient relative to an RT treatment system for the RT treatment to occur where the method comprises:
providing a patient support table;
providing an MR imaging system for acquiring MR images of an imaging volume including a target location of the patient;
the MR imaging system including:
   an MR magnet with gradient coils operable to generate a variable magnetic field to be applied to the patient, the magnet having a cylindrical bore for surrounding the target location of the patient;
   an RF transmit coil arrangement for generating an RF pulse in a transmit stage to be applied to the subject to be imaged such that the subject generates an MR signal in response to the magnetic field and the RF pulse applied;
   a receive coil arrangement for acquiring the MR signal in a receive stage;
   and a signal processing system for receiving the MR signal for carrying out signal processing by which an image is generated;
providing a treatment apparatus for applying a radiation therapy to the target location in the patient while on the support table;
the treatment apparatus having an X-ray imaging system for obtaining an X-ray image of the patient using X-rays;
the treatment apparatus having an X-ray imaging system that shares the coordinate system of the rotational intersection point of the radiation beams;
the MR magnet being mounted for movement from a position remote from the patient on the patient support table to an imaging position for cooperation with the patient on the patient support table for MR imaging; the MR imaging system having a diagnostic scanner table for obtaining an MR image of the patient for RT treatment planning purposes
providing a phantom with fiducial markers embedded within to enable image-based geometric alignment.
using the MR imaging system to obtain a calibration MR image of the phantom with fiducial markers prior to all treatment fractions;
using the X-ray imaging system of the treatment apparatus to obtain a calibration X-ray of the phantom with fiducial markers prior to all treatment fractions;
registering calibration X-ray images taken by the X-ray imaging system relative to calibration MR images taken by the MR imaging system to generate a transformation algorithm required to align the MR coordinate system relative to the X-ray coordinate system;
prior to each day of treatment for a plurality of patients, performing a quality assurance procedure of the transformation algorithm by repeating the calibration MR and X-ray image acquisitions of the phantom with fiducial markers;
prior to each day of treatment updating the transformation algorithm based on the quality assurance results, if necessary;
using the MR imaging system to obtain MR images of a part of the patient to determine the target location of a treatment site in the patient;
using the X-ray imaging system of the treatment apparatus to obtain X-ray images of the part of the patient for use in guiding the RT treatment;
   using an initial MR image data set collected on the diagnostic side of the MR system and an initial CT image data set to generate an RT treatment plan for the patient to be carried out in a plurality of separate treatment steps;
prior to each separate treatment step, obtaining a current MR image of the part of the patient;
and using the transformation algorithm to apply the necessary alignment of the current MR image to guide the RT treatment step.

Thus, by incorporating image guidance immediately before each treatment session, it is possible to determine the exact position of the lesion within each treatment session. Acquiring MR images immediately before RT treatment acts to identify the exact lesion location, and define the correct gantry positions for conformal radiation delivery.

Preferably the transformation algorithm comprises a rigid body transformation matrix.

Preferably the rigid body transformation acts to transfer the MR image in MR imaging coordinates into imaging coordinates of the X-ray imaging system.

Preferably the rigid body transformation acts to align an isocentre of the MR imaging system with an isocenter of the X-ray imaging system.

Preferably the X-ray images taken by the X-ray imaging system are registered relative to MR images taken by the MR imaging system by using a phantom having fiducials arranged to locate the phantom in the image of both modalities.

Preferably the patient support table is movable between an MR imaging location and a RT treatment location.

Preferably the phantom is located in a fixed position on the patient support table when the patient support table moves between an MR imaging location and a RT treatment location.

Preferably the registration is carried out periodically and after completion the transformation algorithm is used for treatments of a plurality of different patients.

Preferably the registration is repeated prior to each treatment step of a patient.

Preferably the patient table is moved in order to re-locate the patient relative to the RT treatment in dependence on the data from the current MR image.

Preferably the patient table is moved relative to the RT treatment in dependence on the X-ray image taken at the time of treatment to provide an initial position and then is moved again in dependence on the data from the current MR image.

Preferably a pre-calibrated geometric distortion correction is also applied to the MR image data set depending on the sequence parameters. The correction is applied to any MR image prior of its use for registration to other MR or x-ray images.

Preferably the current MR image is applied on the image guidance software and registered to the initial MR image to provide a soft-tissue-based transformation matrix required to align the current tumour position at the time of treatment to the initial RT program tumour position to account for any tissue deformations, and tumour position changes.

Preferably the transformation matrix for the MR-to-MR alignment is saved separately.

Preferably CT to CBCT transformation parameters are added to the transformation matrix for the MR-to-MR alignment and this combined transformation matrix is used to adjust the position of the treatment couch so as to bring the patient position to the isocenter of the RT Plan, and ensure that the treatment is delivered accurately based on position of the tumour and other soft tissue structures, not just the bony anatomy alone.

Preferably the method includes measuring the position of the magnet every time it is brought into the imaging position.

Preferably the method includes using any change in the magnet position relative to a magnet position recorded during the initial imaging step in the RT guidance.

Preferably the method includes using information in the MR image file header to confirm the appropriate MR technique was utilized and determine the corresponding distortion correction matrix.

Typically in the treatment of the patient, the patient is assessed in a preliminary step to determine an MR simulation called herein "MR SIM" and CT simulation "CT SIM" imaging for the purposes of treatment planning. Subsequently the actual treatment is carried out on a periodic basis through a series of steps typically on a daily basis which are called herein "Daily MR" which are used in the daily treatment steps. It will be appreciated that the time period can be other than daily as required.

As described herein there is provided a method for patient alignment and quality assurance to enhance the clinical value of MRI-guided radiation therapy procedures, and reduce chances for operator error. The embodiment of this invention includes the overall workflow, a set of calibration tools and corresponding phantoms, a magnet position encoding system, and a software tool to provide coordinate system alignment, image parameter and patient ID screening.

Starting with the commissioning of the MRI-guided RT system, an isocenter alignment calibration step is performed to determine the appropriate shifts required to align the MR coordinate system with the Linac coordinate system.

A calibration procedure is therefore used to determine the vector required to align the MR image set with the Linac coordinate system, and apply this vector directly to the Daily MR images as they are acquired.

This process involves the use of a phantom with an MR and x-ray visible internal structure. The phantom is positioned at the Linac isocenter and imaged using the gantry-mounted x-ray system to produce a CBCT image volume.

The phantom can be of any shape and construction which allows it to be held in fixed position on the patient support table and to include fiducials visible in the MR and X-ray images. These may be the same fiducials or different fiducials with a known spatial interrelation.

Next, the patient couch is reoriented to the MR imaging position with the phantom firmly secured to the tabletop using straps, and the movable MR is brought into MR imaging position. A 3D MR imaging dataset is collected of the phantom at the MR imaging position. The isocenter alignment tool then registers the CBCT and MR images of the phantom, and determines the rigid body transformation matrix required to align the MR isocenter with the CBCT isocenter (which is coincidental with the Linear accelerator beam isocenter). This transformation matrix is then stored in the isocenter alignment tool, such that when subsequent MR imaging dataset are collected on the Linear accelerator, the MR images routed through the isocenter alignment tool automatically have this transformation matrix applied. Ultimately, this tool brings the daily MR image dataset into the same coordinate system as the linear accelerator.

In an MR image distortion correction step, 3D MR image distortion fields will be measured with a dedicated phantom and associated image processing software as part of a standard quality assurance (QA) procedure (i.e., monthly, tri-annual or after any magnet servicing/upgrade). All imaging sequences used for MR Simulation and Treatment Guidance will be investigated. A database of 3D distortion field matrices required for patient image correction will be stored for ulterior use.

The MR alignment and screening tool will identify each MR sequence acquired on the scanner. As an additional utility, the tool can also identify and select the distortion field matrix corresponding to each sequence. The image DICOM header will provide info such as: the (x,y,z) extent of the volume relevant for RT activities. The tool will forward the MR distortion field label info to the treatment planning system (MR Sim/Planning process) and to the Linac (MR Guidance). Depending on the extent of distortions (sequence, FOV, etc.) the image distortion correction (image unwarping) will be applied at the treatment planning station and MR guidance software.

An alternative approach is to correct all MR images right after acquisition and before the MR images reach the MR alignment and filtering tool.

In general, the "MR alignment and screening tool" can also be used to report on the "status of distortion" present in the acquired images: if distortion is required, extent of image volume and prompt distortion correction actions at MR Simulation & Linac treatment guidance.

Overall Image Registration Workflow and MR Image Parameter Screening involves a patient coming in for MR simulation (MR SIM) and CT simulation (CT SIM) imaging for the purposes of treatment planning. The CT image data set is sent directly to the treatment planning station, while the MR data is sent to the MR alignment and screening tool. This tool screens the imaging data to ensure that there is a suitable MR-guidance imaging sequence collected as part of the protocol by cross-referencing against a set of preapproved imaging techniques and parameters. Moreover, this tool also ensures that the MR SIM and MR Guidance data acquired is properly matched with the patient's CT SIM dataset used to create the RT treatment plan. The MR SIM data then is sent to the treatment planning station. In addition to the typical function of the treatment planning station, the MR SIM and MR-guidance is registered to the CT SIM coordinate system, and saved in the CT SIM coordinate system for the purposes of image guidance.

Once treatment planning is complete, the patient comes into the linear accelerator treatment room, and is positioned on the couch using lasers. The linear accelerator image guidance software imports the CT SIM and MR Guidance images, as well as the RT plan. A CBCT image is then collected using the onboard x-ray imaging system. On the Linac, the CT SIM image is then registered to the CBCT image and the coordinate system shift required to align the CT SIM (and corresponding RT Plan) with the Linac coordinate system is stored.

Next, the movable MR is brought into the room and positioned over the patient. The couch is set to a specific position along the superior-inferior axis to ensure that the region-of-interest is within the field-of-view. A check can be performed at this point to ensure that the patient couch is at the correct position relative to the MR and CT Sim. An MR dataset is acquired that is preferably identical to the MR Guidance sequence technique and parameters, which is referred to as the Daily MR scan. The Daily MR scan is sent to the MR isocenter alignment and screening tool where it is screened to ensure that the MR technique is preapproved and that the parameters correspond to the MR Guidance sequence collected during the treatment planning phase. The system can alert the radiation therapist to any discrepancies, and advise how he/she should adjust the data acquisition to ensure that the correct images are acquired. By identifying the sequence used for scanning, this box can also feed info on which image distortion correction transformation is needed to be subsequently applied. The isocenter alignment tool then applies the pre-calibrated transformation matrix to the images by either affecting the DICOM image data or the header information. A pre-calibrated geometric distortion correction may also be applied to the image data set depending on the sequence parameters. Once the Daily MR image set is screened, alignment and possibly rectified (distortion correction), the data is sent to the Linac image guidance software. The process of applying distortion correction could occur in one of two scenarios. In the first scenario, the acquired MR images would be verified and validated by the alignment and screening tool. Based on the sequence used, the corresponding 3D distortion correction transformation matrix would be selected and applied to the images. This would be followed by the application of the MR-to-linac alignment transformation. In the second approach the MR-to-Linac alignment transformation is applied first, and the appropriate distortion correction transformation is selected and applied. The second scenario would require that the MR-Linac alignment shift be accounted for in the 3D distortion correction transformation to account for the updated image coordinates system.

With the aligned/screened Daily MR scan on the image guidance software, the Daily MR is registered to the MR Guidance volume image collected during MR SIM using rigid body automatic or manual registration. This transformation represents the soft-tissue-based transformation required to align the tumour position at the time of treatment to RT Plan tumour position. This step accounts for any tissue deformations, and tumour position changes relative to the information provided in the CT-to-CBCT alignment. This transformation matrix for the MR-to-MR alignment is also be saved separately.

The final step is to add the CT to CBCT transformation parameters to the Daily MR-to-MR SIM transformation parameters, and use this combined transformation matrix to adjust the position of the treatment couch. This brings the patient position to the isocenter of the RT Plan, and ensures that the treatment is delivered accurately based on position of the tumour and other soft tissue structures.

An alternative workflow is to eliminate the CT-to-CBCT registration step, and guide on MR alone. Because the isocenter alignment tool ensures that the daily MR is in the coordinate system of the Linac, it is possible to directly register the MR SIM image to the Daily MR image to align the patient based on MR contrast.

A further component of this system is a method to measure and record the position of the MR in the Linac room every time it is brought into the Linac vault. This position encoding information can be obtained via optical tracking or other positional sensor. This position encoder records the magnet position in the room every time it moves in for imaging. If there has been any change in the magnet position relative to the magnet position recorded during the isocenter alignment calibration step performed in the morning, the relative magnet position shift is added to the calibrated MR-to-Linac transformation matrix. This ensures that the automated isocenter alignment of the Daily MR data includes any shift in the magnet position from the daily morning calibration phase.

The critical advantage of the invention is that it provides a simple approach to calibrating the MR-to-Linac isocenter transformation each day, or before each treatment session, and enables automatic application of this transformation to subsequent MR images collected prior to treatment. This provides a method to ensure that the MR image datasets are in the Linac coordinates to enable expedient and accurate image matching and application of couch shifts to bring the patient position in line with the RT plan using the superior soft tissue contrast of MR.

Another advantage is that the tool provides a means to screen the images to ensure that the MR sequence and parameters are valid for imaged-guided RT, and that a corresponding MR guidance image dataset exists to guide therapy. This ensures both accuracy of the MR-based image-guidance procedure and patient safety, adding a redundant step to avoid operator error in the MR data collection.

A unique aspect of this invention is the overall workflow for aligning the MR coordinate system with the Linac coordinate system. The use of a phantom and calibration phase algorithm is novel, as is the concept to apply the transformation matrix to MR data collected at the time of treatment.

The arrangement disclosed herein provides the ability to perform MR imaging in the same room as external RT without RF interference from the RT system using a specific placement of RF-shielded doors between the two systems that open and close in between radiation therapy and MR imaging. This provides an arrangement aimed to directly combine MR with RT in a hybrid suite.

The arrangement described provides a movable MRI system that can be brought into the room to image the patient before treatment, and then retract the MRI system immediately prior to RT treatment. To implement this movable MRI system with external beam RT treatment requires a novel room configuration and specific shielding requirements to minimize the effects of the linear accelerator on MR image quality, and to ensure optimal operational characteristic of the linear accelerator.

As MR image formation is based on radiofrequency transmission (RF) and detection. MRI systems are typically enclosed within a RF-shielded room to prevent spurious RF signals, external to the MRI system, from interfering with the image acquisition. Depending on the sequence employed, external RF signals create unacceptable artifacts in the image that hinder diagnosis, and degrade the overall image quality. Any electronic devices in the RF-shielded room may be sources of RF, and interfere with image acquisition. Therefore, to combine MRI with RT requires that all devices within the RF-shielded room are RF quiet, i.e., do not generate detectable RF noise. In standard linear accelerators, a number of electronic devices exist that may generate RF noise. One solution to this problem of RF noise involves shutting off all electronic devices in the RT unit during MR imaging, and subsequently powering these devices on for RT treatment. However, this solution has several drawbacks. Thus critical components in the linear accelerator as well as onboard X-ray imaging systems require significant 'warm up' time. Accuracy of the RT system may be compromised by shorter 'warm up' times. Patient throughput is reduced. Repeated power cycling of the electronic components may reduce the overall lifetime of the system. Power up / power down may require additional calibration steps to ensure safe and accurate operation of the device. A better solution involves isolating the critical electronic components of the RT system from the MRI using a set of RF-shield doors between the MRI and RT system that are closed during MR imaging, and opened for radiation treatment.

The basic workflow for external beam RT is as follows:
Patient couch is rotated 180 degrees from the external beam RT system isocenter, and locked into place,
External beam RT vault doors open and movable magnet moves over the patient,
RF shielding doors between the MRI and RT system close,
Patient is imaged with the MRI system,
MRI system is moved back to the diagnostic room, and external beam RT bunker doors close,
RF shielding doors are opened and the patient couch is rotated 180 degrees back to the isocenter of the external beam RT system, and then
Conformal radiation treatment is delivered.

The RF-shielding doors are located between the MR and RT systems, with doors that span the RT vault room and can take any angle for example at an angle perpendicular to the patient or at an angle to pone another such as 45 degrees to the patient so as to come together at an apex. The selection of door orientation is based on space constraints for each site. The RF-shielding doors accounts for the 'toe' portion of the cantilevered patient couch, via a purpose-built notch in the doors that maintains RF-shielding across the patient table.

The arrangement described provides safety control systems integrated into the patient couch to prevent movement when in position for MR imaging to mitigate the possibility of collision between the patient couch and RF shielded doors. Moreover, detectors on the RF doors prevent the doors from closing if there is an object within the path of movement.

This room configuration includes either a separate access to the RT area within the RF-shielded doors, or a separate manual override to enable egress for a person trapped behind the RF-shielded doors via a manual override switch that allows someone the ability to open the doors manually. This manual override system functions even in the event of a complete power failure.

In addition it is required to provide registration between the MR images and the RT images generated for the treatment. An approach for registration is a system where fixed markers are used to automatically map the MR and RT coordinate systems (using the onboard X-ray component of the RT system), which is independent of patient anatomy. These markers are in one embodiment mounted into the patient table, and surrounding patient immobilization devices, and provide a consistent and rigid "global" frame of reference to map between the two coordinate systems.

The embodiment provides an automatic registration system that can be used for mapping between the MR imaging space and RT imaging space without manual intervention. Pre-treatment position verification using a combined MR and RT imaging space coordinate registration ensures accurate delivery of conformal RT.

An array of fiducial markers is embedded into a RT treatment couch top, which is also utilized for pre-treatment MRI acquisition. With this arrangement, the markers are located beneath the patient so as not interfere with the patient position and immobilization. Embedding the makers in the couch top also circumvents the need to affix markers to the surface of the patient and provides a rigid and consistent 'global' reference frame for both MRI and RT imaging acquisitions.

To facilitate registration of the MRI and RT image coordinate systems, the fiducial markers are both MR and X-ray visible and arranged within the field-of-view for both systems. The markers are arranged in a series of unique fiducial frames that are embedded into the patient couch top using special shapes and geometries designed to provide high registration accuracy. A series of different anatomic-based fiducial frames are assigned a particular position in the patient couch top along the inferior/superior (i.e., head-to-toe) direction. Accuracy of the fiducial markers is related to the distance between the fiducial frame and the tissue region of treatment. As such, each of the fiducial frames is specifically designed for the primary anatomical areas typically targeted in RT treatment, e.g.,: 1. head/neck, 2. lung, 3. abdominal, and 4. pelvic. Each fiducial frame consists of several markers in a unique geometry and arrangement that creates a 'signature' for the automatic registration software to identify each frame independently. The MR/X-ray markers can encode the position of the fiducial frame used for each anatomic treatment area, allowing automatic identification of more than one frame with its associated geometry. Markers geometry may also be designed such that if only a subset of the MR/X-ray markers can be identified in the MR and RT imaging system fields-of-view, their location on the frame can still be uniquely identified.

In one embodiment, a bunker is used to contain the RT system on one side, while on the other side of the room is a bunker door that can open, and allow the movable MRI to enter the room. First, the patient is positioned on the patient couch and rotated 180 degrees away from the RT system isocentre. The MRI system moves into the room, and acquire images with the patient in the imaging position, with the fiducial markers embedded into the patient couch top as described above. Once imaging is complete, the movable MRI leaves the room, and the patient is back to the RT system isocentre for X-ray imaging and treatment. In this configuration, the patient position is consistent between both the RT and MRI positions, ensuring that the global fiducial frame is representative of the patient position in both orientations.

A computer algorithm provides an automatic approach to identify each fiducial frame based on the unique marker positions, and to calculate the exact location of the marker frame in three-dimensional space. This fiducial frame represents a "global" coordinate system for registration purposes. The couch-top fiducials are first identified in pre-treatment MR images to establish the global coordinate system. In the next step, the same couch-top is moved to the RT system imaging position, and CBCT, 2D X-ray, or X-ray fluoroscopic images are obtained, in which the same fiducial markers are detected. The automatic computer algorithm registers the global fiducial reference frame between the two imaging modalities and calculates the coordinate mapping between the MRI space and RT space. This allows automatic registration of the MR and RT coordinate systems, without user interaction. However, the software workflow provides a display of the registered MRI and x-ray images for visual confirmation of the alignment of the markers, as well as the alignment of the major anatomical structures. The clinical staff members are able to confirm the success of the automatic registration prior to treatment. The coordinate system transformation matrix can then be used in the RT system to align the gantry isocentre with the pre-treatment MRI position verification, and start treatment.

This arrangement provides the ability to automatically produce the coordinate mapping from a set of MR images to the RT system coordinate system.

Specific advantages may include:
- Enhanced workflow by automating the image registration,
- Fiducial frame is semi-permanently embedded into the patient couch and remain in place indefinitely. (markers do not need to be removed),
- Placement of fiducials in the couch-top ensures a consistent and rigid frame of reference,
- Effectiveness of the fiducial frame not affected by draping, or any other immobilization devices,
- Since the markers are independent of any MR coil and patient anatomy, they are not dependent on coil placement, coil movement or patient positioning,
- Proximity to the tracking volume allows lower average tracking error (i.e., error amplification minimized with a small distance to the fiducial markers as compared to, e.g., having markers out side of the bore that are tracked using an external optical device),
- Fiducial frame is always underneath the patient and does not interfere with patient positioning, and does not require additional preparation time to set up the markers prior to MR imaging or RT treatment.
- Accurate indexing of the patient couch to enable correct patient positioning to ensure that the anatomical region-of-interest is placed over the appropriate patient fiducial frame.
- MR/X-ray encoding markers are used to automatically identify each of the fiducial frames for the corresponding anatomy.
- High spatial accuracy of the patient couch control systems avoids the need for specific 'localizing' MRI sequences, and ensures consistent positioning for X-ray imaging system mounted on the RT unit.
- Placement of MR/X-ray markers in patterns allows identification and localization of the markers when only a subset of markers are captured in the MR and X-ray fields-of-view.
- Marker positioning enables accurate mapping of MR coordinate system with either standard 2D projection X-rays as well as cone-beam CT imaging.
- The ability to support automatic registration without the need to remove the fiducial frame.
- Lower average error since fiducial frame is rigidly embedded into the patient couch top.
- The application of MR and X-ray visible markers embedded into the couch top for alignment of RT treatment.
- MR/X-ray encoding markers can be used to automatically identify the type of fiducial frame (i.e. head/neck or abdominal)
- Placement of MR/X-ray markers in patterns allows identification and localization of the markers when only a subset of markers is captured in the MR and X-ray fields-of-view.
- The use of markers that are both MR and X-ray visible that are embedded in the patient couch top for MR guided RT.
- One solution is to utilize a movable magnet that can be brought into the RT vault for imaging and then retracted prior to radiotherapy procedures. This enables RT that is carried out using the currently available devices, and does not require complex design of hybrid systems that are capable of simultaneous MR and RT procedures.

### BRIEF DESCRIPTION OF THE DRAWINGS

Embodiments of the invention will now be described in conjunction with the accompanying drawings in which:
Figure 1 is a top plan view of an apparatus for radiation treatment of a patient including an MR imaging system showing different positions of the movable magnet.
Figure 2 is a side elevational view of the apparatus of Figure 1 showing the patient support table cooperating with the magnet in the imaging position and also illustrates the patient support table showing the base, toe portion, heel portion, support wheels, motors and couch or table top.
Figure 3 is a top plan view of the apparatus of Figure 1 showing the RF shielding doors.
Figure 4 is a top plan view of the apparatus of Figure 1 showing the RF shielding doors cooperating with a turntable of the patient support table.
Figure 5 is a cross-sectional view along the lines A-A of Figure 4 showing the RF shielding doors.
Figure 6 is a top plan view of an apparatus using the arrangement of Figure 1 in an alternative configuration of the rooms.
Figure 7 is an isometric view of the embodiment of Figure 6 showing the RF shielding doors in the closed position for operation of the MR Imaging system.
Figure 8 is an isometric view of the embodiment of Figure 6 showing the RF shielding doors in the open position with the magnet moved partly away for operation of the Radiation treatment system.
Figure 9 is a top plan view of an apparatus using the arrangement of Figure 1 in a further alternative configuration of the rooms.

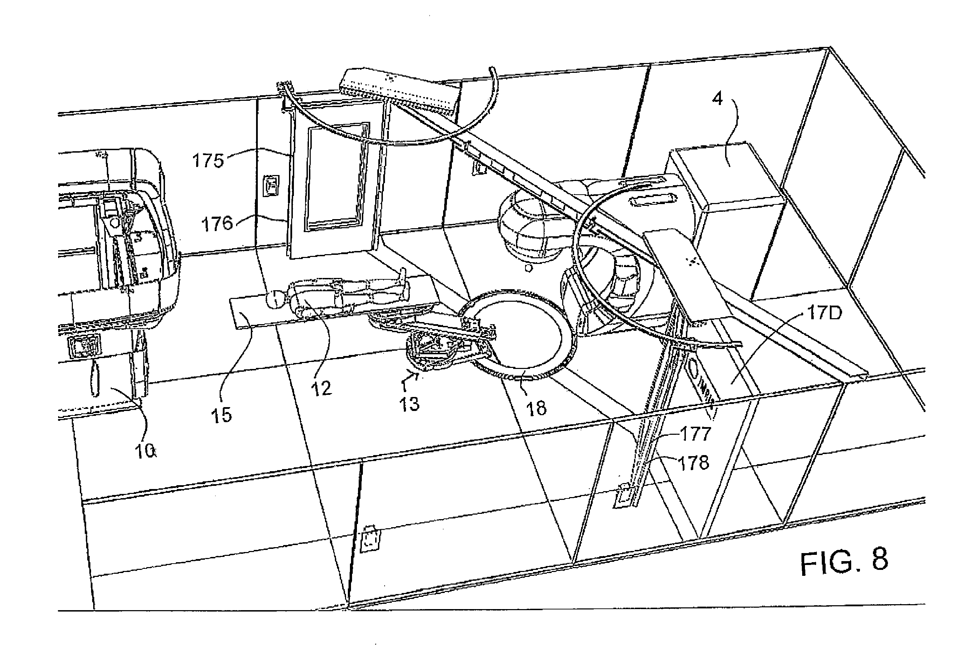

Figure 8 is a flow chart of the automatic registration algorithm.

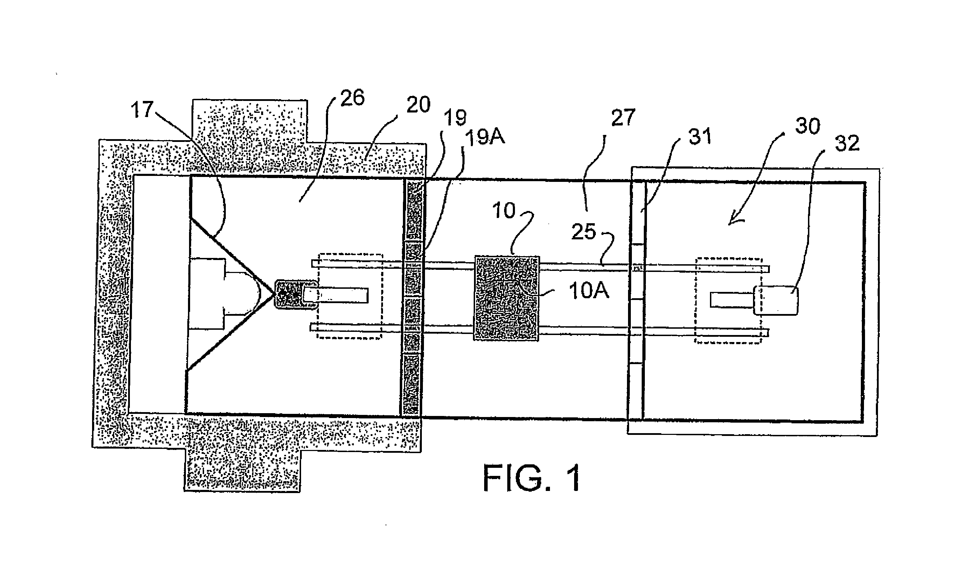

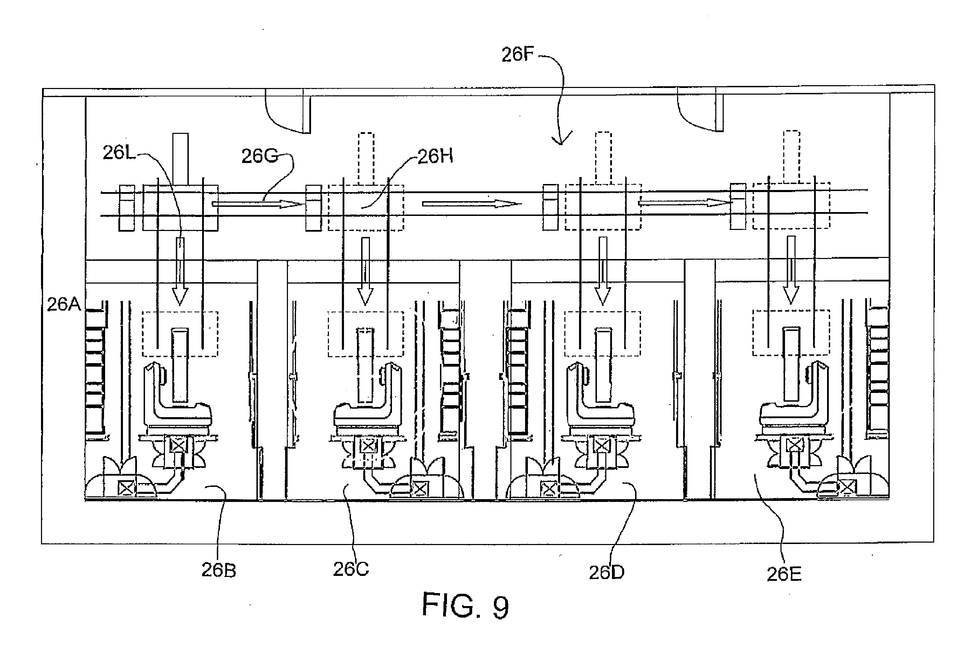

Figure 9 is an isometric view of the table top of Figure 1.

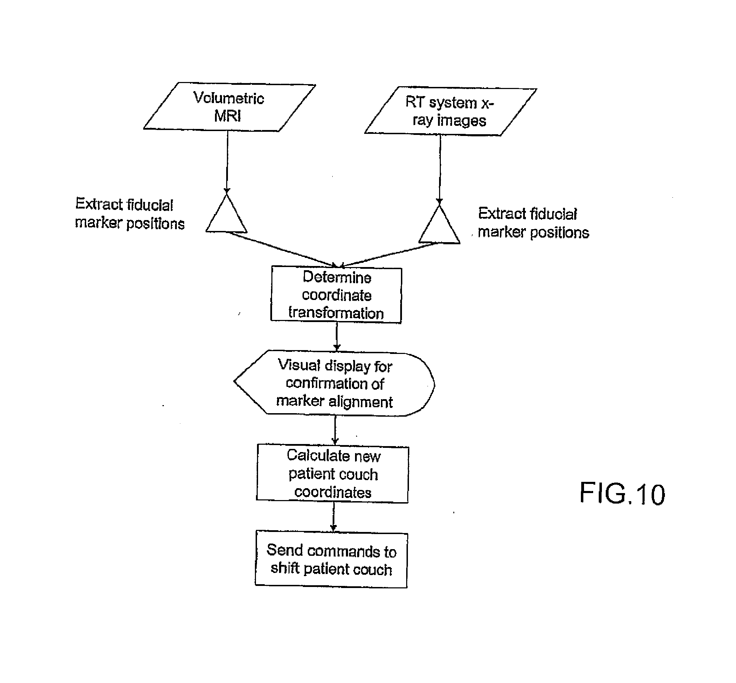

Figure 10 is a flowchart for MRI-guided RT image registration and corresponding couch shifts.

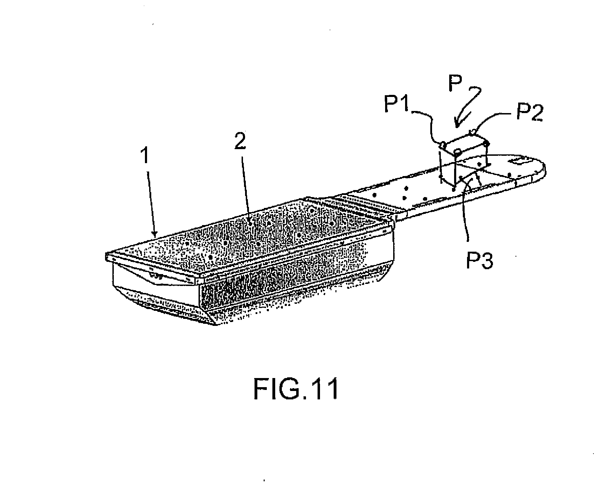

Figure 11 is a diagram demonstrating the MRI-guided RT isocenter alignment procedure

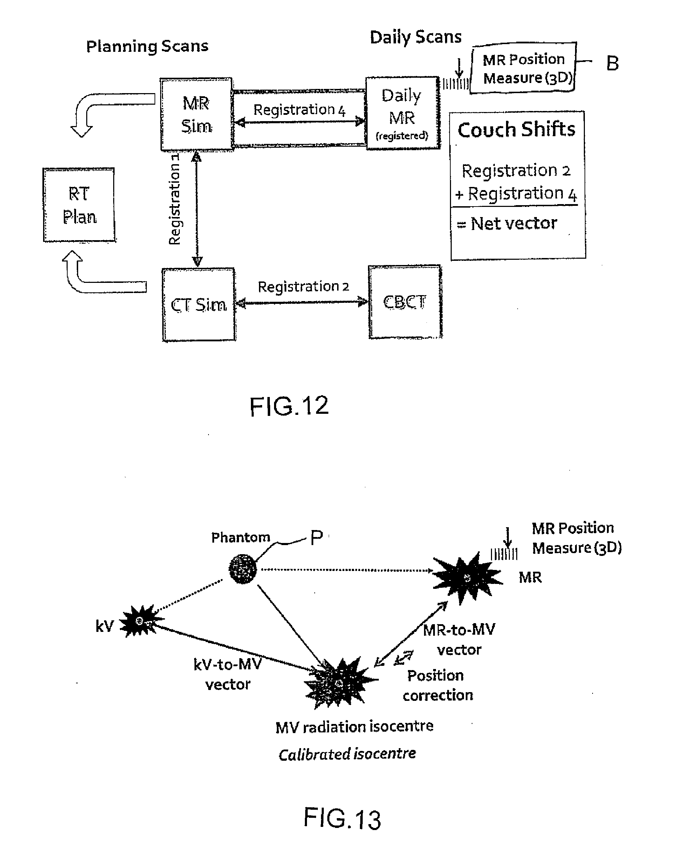

Figure 12 is a dataflow showing the MR isocenter alignment and screening tool position in the RT image-guidance software architecture.

In the drawings like characters of reference indicate corresponding parts in the different figures.

### DETAILED DESCRIPTION

In figures 1 to 5 is shown a schematic diagram of the MRI and external beam RT therapy system within the external beam bunker room configuration. This diagram includes a magnet 10, having a bore 10A into which a patient 12 can be received on a patient couch top 15, which is attached to a patient couch 13.

The movable magnet is carried on a rail system 25 with a support 23 suspended on the rail system. Further details of this construction are available from published US application 2008/0038712 published February 14 2008 assigned to the present assignees, the disclosure of which is incorporated herein by reference.

A suitable radiation therapy system 4 is available from Varian. This can use different radiation including proton beams, brachytherapy or X-ray. The Varian radiation therapy system is capable of delivering therapeutic radiation to the patient using an x-ray beam formed through a multi-leaf collimator to modulate the intensity of the radiation delivered.

The Varian radiation therapy unit also includes a gantry-mounted x-ray imaging system 4A capable of performing cone-beam CT imaging immediately prior to radiation therapy, which enables image-guided radiotherapy. Alternatively, the Varian radiation therapy system is a device capable of delivering brachytherapy. This system also includes an image-guidance system that consisted of an x-ray imaging system. The disclosure of this material is incorporated herein by reference.

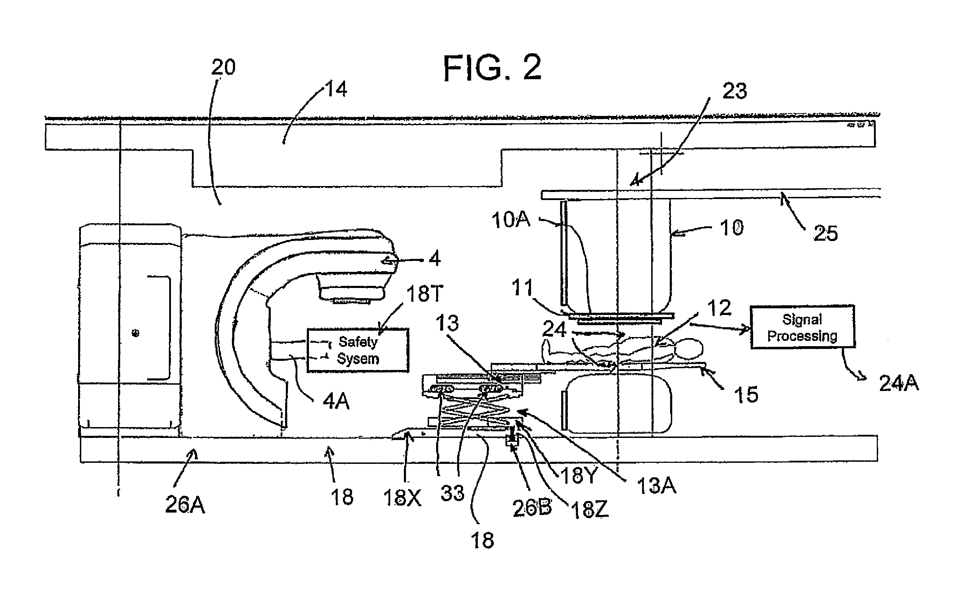

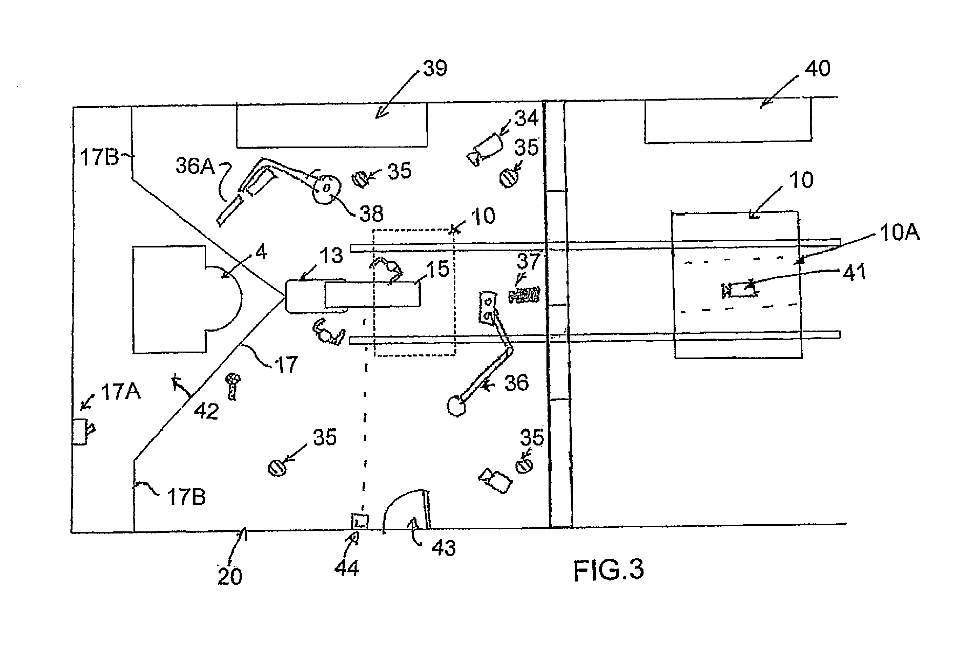

The Patient Handling System or support table is shown in Figure 3, indicated generally at 13 and 15. The patient support table 13 includes a base or pedestal 13A which allows the base to move a patient support portion 15 to required locations in height and in orientation. At the top of the base 13A is mounted the patient support portion 15 in the form of a generally planar body 15 formed of a fiber reinforced plastics material so as to define a surface area sufficient for supporting the patient while lying on the patient support portion. In Figure 1 and 2, the patient and the support structure are rotated 180 degrees from the position of the RT isocentre 16, with the movable MRI in position for imaging. The pedestal 13A is mounted on a turntable 18 for rotation about a vertical axis.

The table can be a multi axis movable or "robot" table of a type also available from Varian. This acts to hold the base stationary while the table top can be moved to any orientation around the base and relative to axes transverse to the base.

As best shown in Figure 2, the table is shown including the base 18, toe portion 18X, heel portion 18Y, support wheels 18Z, motors 33 and couch or table top 15. There are also provided safety control systems 18T at a suitable location relative to the system and integrated into the operation of the patient support table to prevent movement when in position for MR imaging to mitigate the possibility of collision between the patient support table and the RF-shielded door arrangement.

The patient support table 13 is arranged to rotate 180 degrees from the isocentre of the treatment apparatus for cooperation with the MR magnet in MR imaging procedures, and then rotate back to the isocentre for treatment.

The patient support table 13 employs several electronic motors 33 designed to provide different degrees of motion with the motors and controls therefor arranged to avoid interaction of the MRI static magnetic field.

The longitudinal range of motion of the patient support table is extended to provide a safe distance between the static magnetic field of the magnet and positional motors in the patient support table.

The automated system de-energizes the motors 33 once the patient support table is locked in place for MR imaging, and prevents any unsafe actions once locked into place.

The safety system also guarantees that the patient support table is in the appropriate locked position prior to the magnet moving over the patient.

The materials in the table top 15 of the patient support table are both MR compatible and radiolucent to enable both MR and X-ray imaging procedures.

The table top 15 incorporates placeholders for RF coils 24 required for imaging the head and neck or the thorax, abdomen and/or lower pelvic region.

The table top 15 also includes notches at the end of the table top required to maintain a similar function to standard linear accelerator and CT patient table tops.

The table top 15 uses radiolucent and MRI compatible materials in the table top which allows the patient support table to be used in MRI and X-ray imaging systems without degrading the image quality of either system.

The system further includes a receive coil system generally indicated at 24, which receives MR signals generated from the human body in a conventional manner. An RF control system acts to control the transmit body coil 11 and to receive the signals from the receive coil in the general area of 12. When the imaging procedure is complete, the receive coils are attached to the outside of the magnet for transport back to the diagnostic room.

As shown in Figure 1, the external beam bunker room 26 is enclosed within walls 20, ceiling 14 and floor 26A comprised of radiation and RF shield material. The external beam RT room 26 is separated from a diagnostic imaging room 27 by a set of radiation and RF shielded bunker doors 19, so as to prevent radiation exposure to subjects in the adjacent diagnostic room during RT treatment delivery. The external beam bunker doors 19 are open during the imaging procedure and closed during RT delivery.

A second set of RF shield doors 17, situated between the RT and MRI system, are used to isolate the RF generating components of the RT from the MRI system. These doors account for the RF shielding requirements across the turntable portion 18 of the patient support table 13. These doors are closed for MRI procedures and opened for RT delivery. An egress switch 17A is included within the RF-shielded doors to enable a manual override, which allows someone trapped behind the RF doors to open the RF doors even in the event of a power outage.

Following the imaging procedures, the RF shield doors 17 are opened, and the MRI system is moved out of the external beam RT room 26. After the MRI is out of the bunker room, the RF-shielded doors 19 between the MRI and RT systems 17 are opened, and the patient 12 is rotated 180 degrees to the RT position using the patient support table 13. The patient alignment is adjusted based on the imaging results, and the RT beam is delivered from the treatment apparatus 4 to the lesion via the conventional collimator and rotating support.

In Figures 1 and 2 the three-room configuration is displayed, with the rooms for external beam treatment 26, diagnostic imaging 27 and brachytherapy 30. RF and radiation-shielded doors 31 are placed between the diagnostic room 27 and brachytherapy bunker 30. The patient is positioned on the brachytherapy support table 32, which can allow the MRI system to move over the patient, and also has the necessary patient supports for brachytherapy procedures.

Figure 3 provides a sketch of the complete room configuration for the three-room configuration with brachytherapy. In addition to the previous figures, components such as the two patient cameras 34, MR-compatible pot lights 35, IR tracking camera system 36 are positioned within the system room configuration. Based on the radiation damage that could be incurred at certain points in the room design, the third patient camera 37 is a radiation hardened camera that has electrical components not seriously affected by radiation exposure. The egress switch 17A is also included in Figure 3. LCD monitors 36A are positioned at approximately position 38 to be easily visible by the clinical staff in the room. The bunker room also includes cabinets 39 for storage of immobilization and other radiotherapy devices. The MR coils used in the imaging procedures are located in the diagnostic room storage cabinets 40. To enable continuous patient monitoring, an MR-compatible camera 41 is mounted inside the bore 10A of the movable MRI system 10. Speakers and a microphone are installed at approximately position 42, which enable continuous communication with the patient when the clinical staff are outside of the room. A personnel access sliding door 43 is designed into the room to provide direct access to the bunker room. The room also has a laser system 44 mounted on the bunker room wall 20, that allows the clinical staff to accurately position the patient prior to MR imaging or radiation therapy delivery.

The apparatus for radiation therapy of a patient therefore includes the patient support table 13, a magnetic resonance imaging system for acquiring MR images of an imaging volume including the target location of the patient and the radiation treatment apparatus 4.

The MR imaging system includes the MR magnet 10 with gradient coil 10A operable to generate a variable magnetic field to be applied to the patient, the magnet having a cylindrical bore 10A for surrounding the target location of the patient. The imaging system further includes the RF transmit coil arrangement 11 for generating an RF pulse in a transmit stage to be applied to the subject to be imaged such that the subject generates an MR signal in response to the magnetic field and the RF pulse applied. The signal is picked up by the receive coil arrangement 24 for acquiring the MR signal in a receive stage. The received signal is transmitted to a signal processing system 24A for receiving the MR signal for carrying out signal processing by which an image is generated.

The beam or brachytherapy treatment apparatus 4 is arranged for applying a radiation therapy to a target location in the patient on the support table.

The patient support table 15 and the treatment apparatus 4 are mounted in a treatment room having an opening 19A which includes a radiation shielded door arrangement 19 defined by two doors movable at right angles to the walls to a center closed position operable to close the opening 19A.

The MR magnet 10 is mounted for movement from a position exterior of the radiation shielded door arrangement 19 into or through the opening 19A to an imaging position for cooperation with the patient on the patient support table 15 for MR imaging.

The RT system 4 is separated from the magnet during imaging by the RF-shielded door arrangement 17 in the treatment room arranged to be movable between a closed position, separating at least part of the treatment apparatus from the magnet when in the imaging position, and an open position in which the treatment apparatus is accessible to the patient on the patient support table for radiation therapy.

The RF-shielded door arrangement 17 is arranged in the closed position to isolate RF noise critical electronic components of the treatment apparatus 4 from the magnet 10 and receive coil arrangement 24 while allowing the critical electronic components of the treatment apparatus 4 to remain active without shutdown. This is necessary to avoid unacceptable delays which would be caused by a shut down of the system and restarting.

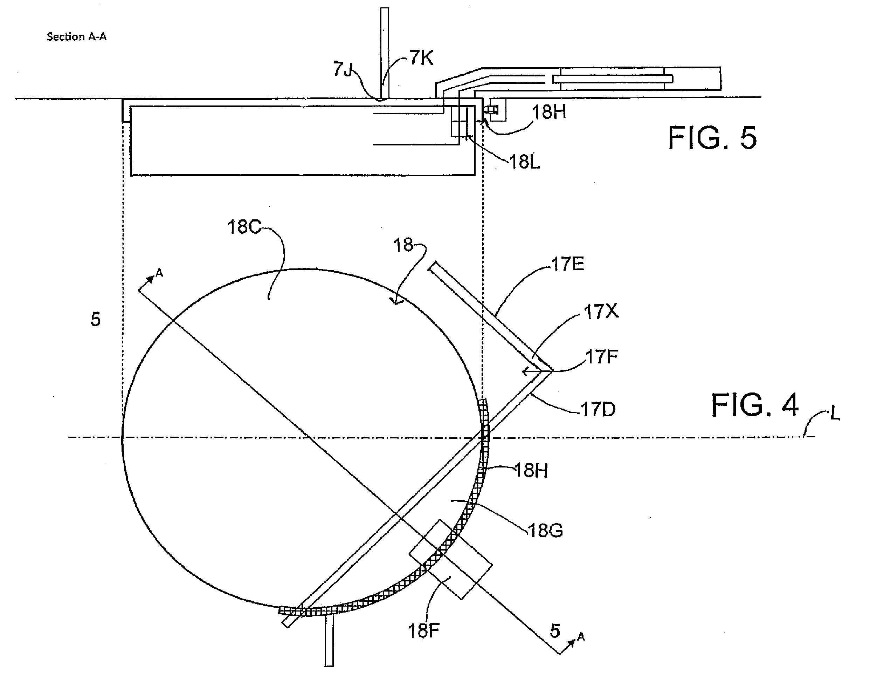

The RF-shielded door arrangement 17 includes a suitable conductive layer typically formed of copper which halts and RF signals at the conductor. As shown in Figures 3 and 4, the RF-shielded door arrangement includes doors or components 17 which together span the treatment room and which extend at an inclined angle to the patient support table. Thus there is provided an RF shielded wall portion 17B arranged on each side of the treatment apparatus at an angle to a longitudinal axis of the patient support table which can remain fixed and in position partly spanning the room. Each wall portion 17B cooperates with a respective RF shielded door 17 which can move across the treatment room along its respective wall portion from the inner edge of the respective wall portion so that the doors meet at the middle and form a continuous RF conductive shield. The position of the wall portions 17B is such that the table can rotate around the vertical central axis to take up its two positions while moving inside the inner edges 17C of the wall portions.

As best shown in Figure 4, the doors 17D and 17E meet at a point 17F which is symmetrical to the center line L of the system, and both doors extend across the turntable 18.. Thus the RF-shielded door 17D extends across a part 18 of the base so as to exclude the main part 18C of the base from the area containing the MR magnet. A portion of the base 18 as indicated at 18G is arranged inside the area of the magnet with the toe portion of the couch.

Thus the RF-shielding door arrangement 17 includes a RF-shielding inflatable bladder 7J in a bottom edge 7K of the doors that maintains RF-shielding across the base 18 of the patient support table. The surface baseplate of the turntable 18 is formed of a conductive material and there is provided a movable contactor 18H in the form of an inflatable bladder connected to the RF shield for engaging the turntable. In this way the continuous conductive shield communicates through the turntable 18C and the door 17D, mounting the parts of the table which are inside the zone of the magnet so that they are all shielded from the RF signals generated by the quiescent but active treatment system in the zone behind the doors 17.

The two parts 17D, 17E of the doors come together at an apex and are inclined to a longitudinal axis of the room so as to enclose the treatment system 4 on the left of the doors within the triangular shape defined by the two doors.

Cables for providing control signals to the patient support table pass through a gland at the part of the RF shield defined by the portion 18G of the turntable. In order to ensure that signals generated in the cables by the RF fields within the magnet zone are not communicated through to the zone of the treatment apparatus and vice versa, an RF filter system 18L is connected in the cables to filter the signals.

On the patient support table some of the control elements are operated by fiber optic cables 18M which pass through the RF shield at the base portion 18G.

There are provided one of more detectors 17X on the RF-shielded door arrangement to prevent the door arrangement from closing to the position 17F if there is an object within the path of movement.

In one arrangement, there are provided three rooms including a first room for the Radiation treatment system 26, a third room 30 for the brachytherapy system combined with a second intermediate diagnostic room 27 in the middle to provide a three-room solution that provides image-guidance for both external beam and brachytherapy RT procedures using the same movable MRI magnet.

In Figures 1 and 2 there are provided two separate rooms where the first is a radiation-shielded "bunker" that houses the treatment apparatus and the second room houses the MR magnet for basic diagnostic procedures.

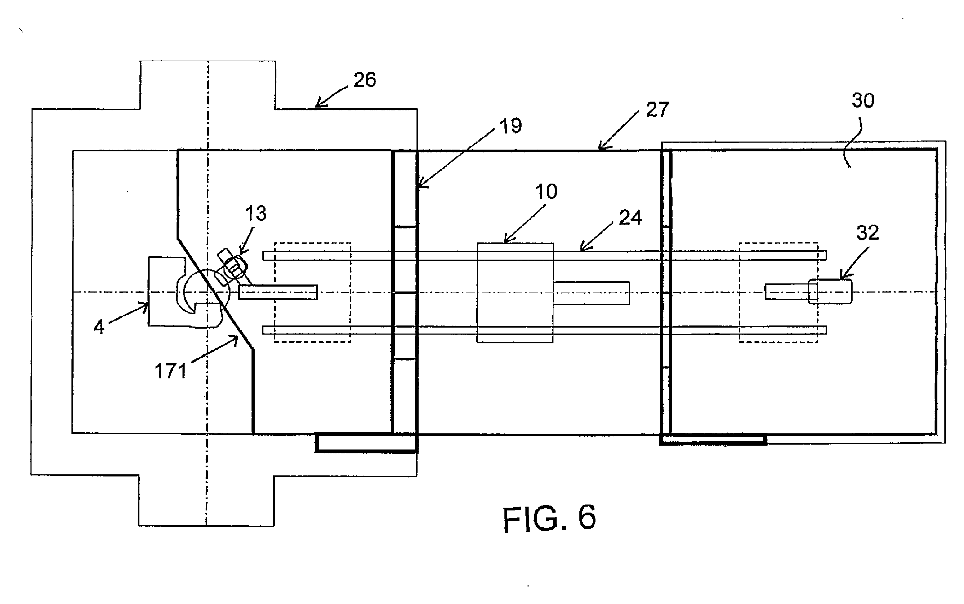

Turning now to Figures 6, 7 and 8, substantially the same arrangement as that of Figures 1 and 2 is shown. In this arrangement the robotic table 13 available from Varian which rotates around the turntable 18 is shown in more detail.

In this arrangement the RF shielding door arrangement indicated generally at 171 is different in that it includes a part 172 extending fully across the turntable 18 to a position 173, 174 on either side of the turntable 18. The treatment apparatus 4 includes a head 4B rotatable about an axis longitudinal of the treatment room.

The head 4B, as is well known in these systems rotates around the longitudinal axis to direct the radiation to the treatment location from different angles around the patient.

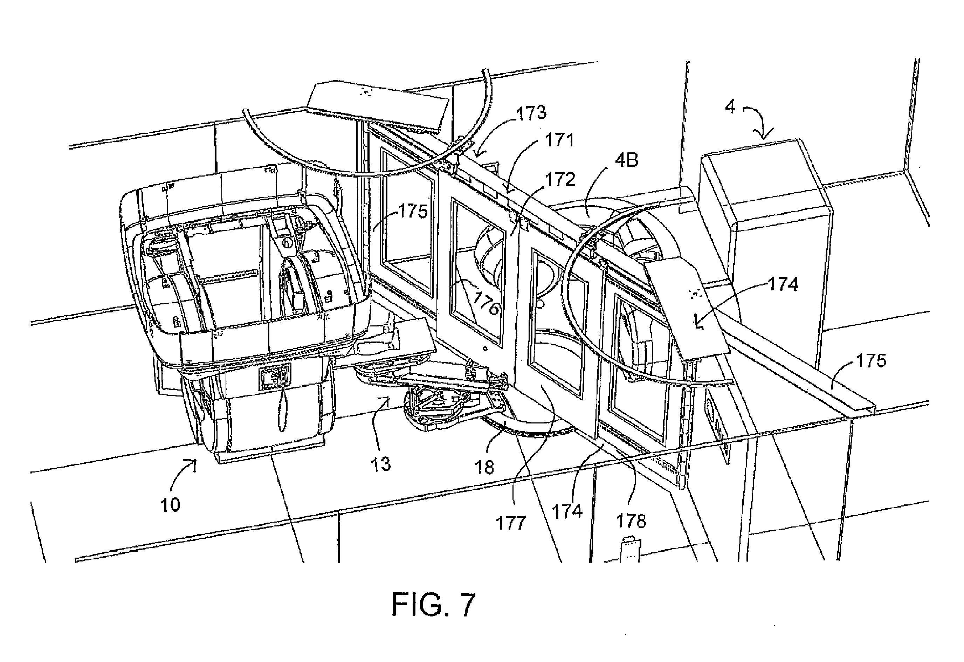

In this arrangement use is made of the position of the head where the head 4B is turned 90 degrees to one side of the patient support table. This enables the door part 172 to pass generally diagonally across the turntable 18 with the head on the side of the door 172 remote from the magnet. The door 172 is formed of a conductive material to create the required RF shield. The bottom of the door cooperates with a band 174 of conductive material across the floor and similarly the top of the door 1'72 cooperates with a band 175 across the ceiling. Using a bi-fold or sliding door arrangements made up of door panels 175, 176, 1'77 and 178 allows the door arrangement to fold back against the wall on one side and against a wall portion 17D on the other side. In this way the door arrangement can be moved to both positions without difficulty, without interfering with the patient on the table 13, while leaving the patient and table 13 wholly exposed for the magnet 10 as shown in Figure 7 and while leaving the area open as shown in Figure 8 for the treatment process.

Turning now to the separate embodiment shown in Figure 9, the room configuration includes a row 26A of rooms 26B to 26E each having a respective treatment apparatus, a respective patient support table and a respective radiation shielded door arrangement and the magnet is arranged to move along a path or passageway 26F outside the row of radiation shielded door arrangements. From this path, the magnet is arranged to move linearly along the path 26G outside the row of radiation shielded door arrangements to each room in turn as required. From this position the magnet rotates about a vertical axis 26H at each room to cooperate with the patient support table therein. In addition to the rotation, the magnet may also move in a direction 26L at right angles to the path 26G to enter the opening of the room concerned. However in some arrangements the magnet may not be able to move in the direction 26L and the cooperation between the table and the magnet is achieved by movement of the table alone.

Turning now to Figures 10 and 11, an arrangement is provided for automatic registration of MR images with X-ray images obtained by the treatment apparatus without manual input. It will be appreciated that the treatment apparatus includes an X-ray imaging system used to plan the treatment and it is necessary to register the MR images with the treatment image for planning and control of the treatment. For this purpose, an array 1 of MR and X-ray visible fiducial markers 2 is embedded into a patient support couch top of the patient support table. The markers are located beneath the patient so as not interfere with the patient position and immobilization. The fiducial markers are embedded into the couch top for alignment of the RT treatment. The array 1 is arranged such that the encoding markers can be used to automatically identify the type of fiducial frame (i.e. head/neck or abdominal). That is the array contains patterns of markers which are tailored to the frame to be used with the table. This pattern can then be recognized by the imaging system to determine the frame under use without necessity for manual input of this information. In addition, placement of MR/X-ray markers is arranged in patterns to allow identification and localization of the markers when only a subset of markers is captured in the MR and X-ray fields-of-view.

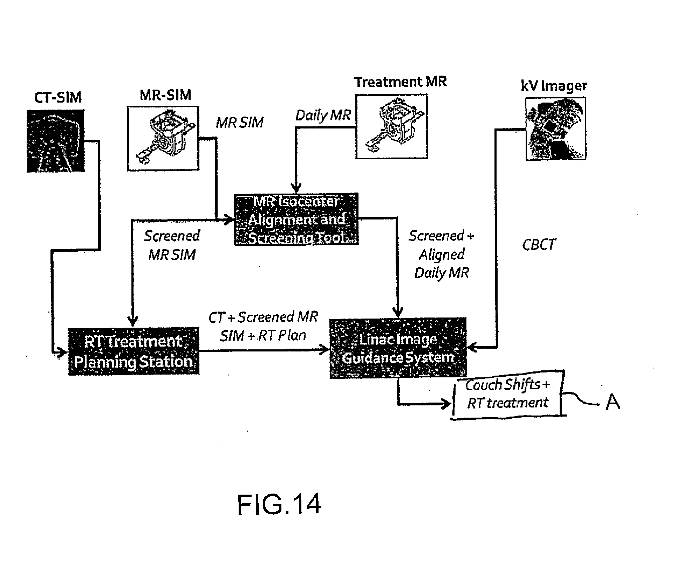

Turning now to Figures 12, 13 and 14 and particularly Figure 14, the method for treating a patient with radiation therapy includes using the diagnostic side of the MR imaging system to obtain MR images of a part of the patient, for the purposes of MR SIM, to determine the target location of a treatment site in the patient and using a separate X-ray imaging system (not depict in the figures) to obtain X-ray images of the part of the patient for use in guiding the RT treatment (CT SIM). These are used at the RT treatment planning station to generate an RT treatment plan for the patient to be carried out in a plurality of separate treatment steps by the Linac image guidance system.

In an MR Isocenter Alignment and Screening Tool, X-ray images of a phantom taken by the X-ray imaging system are registered relative to MR images taken by the MR imaging system to generate a transformation algorithm required to align the MR images of the part of the patient relative to the X-ray images of the part of the patient. In addition the tool will also identify and select the corresponding MR distortion correction matrix corresponding to each MR image sequence. And, send the matrix information to treatment planning system (MR Sim) and Linac image guidance system (MR Guidance), or apply the distortion correction immediately. This set of distortion correction matrices are periodically acquired (e.g., monthly QA) and stored for each image sequence used for RT Sim/guidance.

Prior to each separate treatment step in the regimen determined in the planning stage, a daily or current MR image of the part of the patient is obtained and after transformation using the transformation algorithm of the MR-to-RT coordinates, the data from the current MR image is used in guiding the RT treatment step in the Linac Image Guidance system.

The transformation algorithm comprises a rigid body transformation matrix which acts to transfer the MR image in MR imaging coordinates into imaging coordinates of the X-ray imaging system, which are coincidental to the treatment beam coordinates, and to align the isocentre of the MR imaging system with the isocenter of the X-ray imaging system.

The X-ray images taken by the X-ray imaging system are registered relative to MR images taken by the MR imaging system by using a phantom P (Figures 11 and 12) having fiducials P1 and P2 arranged to locate the phantom in the image of both modalities. As shown in Figure 11, the patient support table is movable between an MR imaging location and a RT treatment location. The phantom P is located in a fixed position on the patient support table by a mount P3 when the patient support table moves between an MR imaging location and a RT treatment location. The registration is carried out periodically and after completion the transformation algorithm is used for treatments of a plurality of different patients. Each day before the first daily treatment, the registration is repeated to generate an updated transformation algorithm that can be applied to all MR images for all patients treated that day.

The patient table is adjusted in its fine positioning by the Guidance system which acts on actuators A in the couch in order to re-locate the patient relative to the RT treatment in dependence on the data from the current MR image. The patient table is moved relative to the RT treatment in dependence on the current or daily X-ray image taken at the time of treatment to provide an initial position and then is moved again in dependence on the data from the current or daily MR image.

As shown in Figure 13 the position of the magnet is detected every time it is brought into the imaging position using a 3D position sensor B so that any change in the magnet position relative to a magnet position recorded during the initial imaging step is used in the RT guidance.

The basic workflow in Figure 14 involves a patient coming in for MR simulation (MR SIM) and CT simulation (CT SIM) imaging for the purposes of treatment planning. The CT image data set is sent directly to the treatment planning station, while the MR data is sent to the MR alignment and screening tool. This tool screens the imaging data to ensure that there is a suitable MR-guidance imaging sequence collected as part of the protocol by cross-referencing against a set of preapproved imaging techniques and parameters. Moreover, this tool also ensures that the MR SIM and MR Guidance data acquired is properly matched with the patient's CT SIM dataset used to create the RT treatment plan. The MR SIM data then is sent to the treatment planning station. In addition to the typical function of the treatment planning station, the MR SIM and MR-guidance is registered to the CT SIM coordinate system, and saved in the CT SIM coordinate system for the purposes of image guidance. The tool can also report on the MR image distortion status for each MR data set acquired on the MR scanner.

Once treatment planning is complete, the patient comes into the linear accelerator bunker, and is positioned on the couch using lasers. The linear accelerator image guidance software imports the CT SIM and MR Guidance images, as well as the RT plan. A CBCT image is then collected using the onboard x-ray imaging system. On the Linac, the CT SIM image is then registered to the CBCT image and the coordinate system shift required to align the CT SIM (and corresponding RT Plan) with the Linac coordinate system is stored.

Next, the movable MR is brought into the room and positioned over the patient. An MR dataset is acquired that is preferably identical to the MR Guidance sequence technique and parameters, which is referred to as the Daily MR scan. The Daily MR scan is sent to the MR isocenter alignment and screening tool where it is screened to ensure that the MR technique is preapproved and that the parameters correspond to the MR Guidance sequence collected during the treatment planning phase. Moreover, the MR image distortion is also evaluated and corrected, if correction is necessary. The system can alert the radiation therapist to any discrepancies, and advise how he/she should adjust the data acquisition to ensure that the correct images are acquired. The isocenter alignment tool then applies the pre-calibrated transformation matrix to the images by either affecting the pixel data or the DICOM header information. A pre-calibrated geometric distortion correction may also be applied to the image data set depending on the sequence parameters. Once the Daily MR image set is screened, alignment and possibly rectified (distortion correction), the data is sent to the Linac image guidance software.

With the aligned/screened Daily MR scan on the image guidance software, the Daily MR is registered to the MR Guidance volume image collected during MR SIM using rigid body automatic or manual registration. This transformation represents the soft-tissue-based transformation required to align the tumour position at the time of treatment to RT Plan tumour position. This step accounts for any tissue deformations, and tumour position changes relative to the bony anatomy (which is accounted for with the CT-to-CBCT alignment). This transformation matrix for the MR-to-MR alignment is also be saved separately.

The final step is to add the CT to CBCT transformation parameters to the Daily MR-to-MR SIM transformation parameters, and use this combined transformation matrix to adjust the position of the treatment couch. This brings the patient position to the isocenter of the RT Plan and ensures that the treatment is delivered accurately based on position of the tumour and other soft tissue structures, not just the bony anatomy alone.

## Claims

1. An apparatus for treating a patient with radiation therapy comprising:
a patient support table;
an MR imaging system for acquiring MR images of an imaging volume including a target location of the patient;
the MR imaging system including:
an MR magnet with gradient coil operable to generate a variable magnetic field to be applied to the patient, the magnet having a cylindrical bore for surrounding the target location of the patient;
an RF transmit coil arrangement for generating an RF pulse in a transmit stage to be applied to the subject to be imaged such that the subject generates an MR signal in response to the magnetic field and the RF pulse applied;
a receive coil arrangement for acquiring the MR signal in a receive stage;
and a signal processing system for receiving the MR signal for carrying out signal processing by which an image is generated;
a treatment apparatus for applying a radiation therapy to the target location in the patient while on the support table;
the treatment apparatus having an X-ray imaging system for obtaining an X-ray image of the patient using X-rays;
the MR magnet being mounted for movement from a position remote from the patient on the patient support table to an imaging position for cooperation with the patient on the patient support table for MR imaging;
the MR imaging system being arranged to obtain MR images of a part of the patient to determine the target location of a treatment site in the patient;
the X-ray imaging system of the treatment apparatus being arranged to obtain X-ray images of the part of the patient for use in guiding the RT treatment;
means to generate from an initial MR image and an initial X-ray image an RT program for the patient to be carried out in a plurality of separate treatment steps;
means for registering X-ray images taken by the X-ray imaging system relative to MR images taken by the MR imaging system to generate a transformation algorithm required to align the MR images of the part of the patient relative to the X-ray images of the part of the patient;
means for obtaining, prior to each separate treatment step, a current MR image of the part of the patient;
and means for using the transformation algorithm to use data from the current MR image in guiding the RT treatment step.

2. The apparatus according to claim 1 wherein the MR imaging system is arranged to obtain a calibration MR image of the phantom with fiducial markers prior to all treatment fractions, wherein the X-ray imaging system of the treatment apparatus is used to obtain a calibration X-ray of the phantom with fiducial markers prior to all treatment fractions and wherein the calibration X-ray images taken by the X-ray imaging system are registered relative to calibration MR images taken by the MR imaging system to generate the transformation algorithm required to align the MR coordinate system relative to the X-ray coordinate system.

3. The apparatus according to claim 1 or 2 including means for performing prior to each day of treatment for a plurality of patients, a quality assurance procedure of the transformation algorithm by repeating the calibration MR and X-ray image acquisitions and updating the transformation algorithm based on the quality assurance results.

4. The apparatus according to any preceding claim wherein the transformation algorithm comprises a rigid body transformation matrix which acts to transfer the MR image in MR imaging coordinates into imaging coordinates of the X-ray imaging system.

5. The apparatus according to claim 4 arranged wherein the rigid body transformation acts to align an isocentre of the MR imaging system with an isocenter of the X-ray imaging system.

6. The apparatus according to any preceding claim wherein the X-ray images taken by the X-ray imaging system are registered relative to MR images taken by the MR imaging system by using a phantom having fiducials arranged to locate the phantom in the image of both modalities.

7. The apparatus according to claim 6 arranged wherein the phantom is located in a fixed position on the patient support table when the patient support table moves between an MR imaging location and a RT treatment location.

8. The apparatus according to any preceding claim arranged wherein the registration is carried out periodically and after completion the transformation algorithm is used for treatments of a plurality of different patients.

9. The apparatus according to any preceding claim arranged wherein the registration is repeated prior to each treatment step of a patient.

10. The apparatus according to any preceding claim wherein the patient table is arranged to be moved in order to re-locate the patient relative to the RT treatment in dependence on the data from the current MR image.

11. The apparatus according to claim 10 wherein the patient table is arranged to be moved relative to the RT treatment in dependence on the X-ray image taken at the time of treatment to provide an initial position and then is moved again in dependence on the data from the current MR image.

12. The apparatus according to any preceding claim arranged for applying a pre-calibrated geometric distortion correction matrix to the image data set depending on sequence parameters of the MR imaging system.

13. The apparatus according to claim 12 arranged for using information in a file header of the MR image to confirm that an appropriate MR technique was utilized and to determine the distortion correction matrix.

14. The apparatus according to any preceding claim arranged wherein the current MR image is registered to an initial MR image to provide a soft-tissue-based MR transformation matrix required to align the current tumour position at the time of treatment to the initial RT program tumour position to account for any tissue deformations and tumour position changes relative to the bony anatomy.

15. The apparatus according to claim 14 arranged for adding a CT to CBCT transformation matrix to the MR transformation matrix and for using this combined transformation matrix to adjust the position of the treatment couch so as to bring the patient position to the isocenter of the RT program, and for ensuring that the treatment is delivered accurately based on position of the tumour and other soft tissue structures, not just the bony anatomy alone.

16. The apparatus according to any preceding claim arranged for measuring the position of the magnet every time it is brought into the imaging position and using any change in the magnet position relative to a magnet position recorded during an initial imaging step in the RT guidance.
